# EUROPEAN PATENT SPECIFICATION

(11) **EP 2 259 765 B1**
(45) Date of publication and mention of the grant of the patent: **15.08.2012**
(21) Application number: 09730409.1
(22) Date of filing: 08.04.2009
(51) Int. Cl.: A61K 8/06, A61K 8/898, A61Q 19/08

(54) **COSMETIC METHOD FOR SMOOTHING WRINKLES AND FINE LINES USING AN INTERPOLYMER**
KOSMETISCHES VERFAHREN ZUR GLÄTTUNG VON FALTEN UND FEINEN LINIEN MITHILFE EINES INTERPOLYMERS
PROCÉDÉ COSMÉTIQUE POUR LISSER LES RIDES ET LES RIDULES UTILISANT UN INTERPOLYMÈRE

(30) Priority: 09.04.2008 FR 0852384
(43) Date of publication of application: 15.12.2010
(73) Proprietor: Chanel Parfums Beauté, 92521 Neuilly sur Seine Cedex (FR)
(72) Inventor: BRAULT, Delphine, F-60300 Senlis (FR); QUEVAUVILLER, Florence, F-78400 Chatou (FR); FRICK, Régine, F-75017 Paris (FR)
(74) Representative: Renard, Emmanuelle
(86) International application number: PCT/EP2009/054244
(87) International publication number: WO 2009/124976

(56) References cited:
- US-A1- 2007 104 677
- DISCLOSED ANONYMOUSLY: "Use of MicroMarimo TiO2 particles in cosmetic compositions" RESEARCH DISCLOSURE, MASON PUBLICATIONS, HAMPSHIRE, GB, vol. 517, no. 36, 1 May 2007 (2007-05-01), page 498, XP007137293 ISSN: 0374-4353

## Description

The present invention relates to a cosmetic method for tightening the skin and/or smoothing wrinkles and fine lines by a tightening effect, comprising the application to the skin of a composition in the form of a water-in-oil emulsion containing, in a physiologically acceptable medium, an interpolymer comprising at least one polyurethane and at least one copolymer of vinylpyrrolidone and a silicone monomer.

Numerous research studies have been undertaken over the past few years in order to develop cosmetic compositions which confer a younger appearance on the skin. These compositions generally contain one or more active agents of a synthetic or plant origin, which are capable, for example, of stimulating the cellular mechanisms involved in the synthesis of collagen, elastin or hyaluronic acid which contribute to the firmness and to the elasticity of the skin, or of strengthening the dermo-epidermal junction.

The disadvantage of these active agents is that the effects which they produce on the skin can only be observed after a certain period. The antiageing compositions available on the market therefore also include in general pigments which produce on the skin optical effects which make it possible to mask imperfections (soft-focus pigments) and/or morphosculptural effects, as well as so-called tightening agents, whose role is to tighten the features and visibly and instantly smooth the wrinkles.

It is however inadvisable to formulate these prior art tightening agents in the presence of oil since it has been observed that oils cause a very marked reduction in, or even the disappearance of, the tightening effect. The antiageing compositions having a tightening effect are therefore instead formulated in the form of gel-like aqueous compositions.

Now, the presence of an oily phase in an antiageing cosmetic composition is often desirable, whether in order to confer an emollient character on the composition and to form at the surface of the skin an occlusive film which avoids it becoming dry and the subsequent appearance of fine dehydration lines, or in order to transport various fat-soluble topical active agents, such as vitamins A and E, in particular, which are particularly useful in anti-wrinkle compositions.

In addition, the presence of an oily phase is generally necessary for the production of antiageing compositions in the form of foundations. Indeed, the latter are generally formulated in the form of emulsions in a continuous oily phase which make it possible to have a better staying power of the makeup, that is to say a better resistance to water and to perspiration.

One of the solutions proposed in the prior art for formulating tightening agents in the presence of oil has consisted in increasing the concentration of tightening agent present in the aqueous phase, with the risk however of conferring an unacceptable tacky character on the composition. Another solution has consisted in combining the tightening agent with compounds which strengthen the tightening effect, in particular ionic amphiphilic polymers (EP-1 419 763), elastomeric film-forming polymers (FR-2 872 410), ethylenic block polymers (WO 2005/30158) or hydroxyalkylated derivatives of urea (WO 2005/56491). However, the compositions obtained are predominantly compositions containing a continuous aqueous phase and the percentage of tightening agent remains high (more than 6% in general). Another solution has also consisted in using, as tightening agents, hybrid polymers consisting of a network of interpenetrating polymers (FR-2 843 025).

The need remains nevertheless to have available a cosmetic composition having a tightening effect, containing an oily phase which may contain more than 30%, or even more than 40%, by weight of oils relative to the total weight of the composition, which composition exhibits on the skin a tangible, immediate and lasting tightening effect, even at a content of tightening agent of less than 1% by weight.

This need is satisfied according to the present invention by a composition in the form of a water-in-oil emulsion containing a particular hydrogel, which is in particular marketed by the company HYDROMER under the trade name Aquamere^{®} S2011 as film-forming agent for care and makeup compositions for the skin.

It has already been suggested to use this hydrogel for increasing the SPF of anti-sun compositions, in particular containing avobenzone (US-6,436,377). In addition, the document US 2007/104677 discloses a facial cosmetic composition in the form of a silicone-in-water (Si/W) or water-in-silicone-in-water (W/Si/W) emulsion containing volatile silicones as well as polymers, such as the abovementioned hydrogel, forming on the skin an elastic film having a tightening effect.

On the other hand, to the knowledge of the applicant, it has never yet been proposed to use the abovementioned hydrogel in a cosmetic composition in the form of a W/O, in particular W/Si, emulsion for smoothing wrinkles and it is to the applicant's credit to have demonstrated that this material makes it possible, unlike most of the known tightening agents, to confer a tangible and lasting tightening effect on the skin, although formulated in the presence of a large quantity of oil.

The subject of the present invention is thus a cosmetic method for tightening the skin and/or smoothing wrinkles and fine lines by a tightening effect, comprising the application, to the skin, of a composition in the form of a water-in-oil emulsion containing, in a physiologically acceptable medium, an interpolymer comprising at least one polyurethane and at least one copolymer of vinylpyrrolidone and a silicone monomer.

The interpolymer used according to the invention may be obtained in general according to a method similar to that described in Patent US-4,642,267, that is to say according to a method comprising the mixing of the copolymer of vinylpyrrolidone and polyurethane in a solvent phase or in the molten state, optionally the addition of a cross-linking agent to the mixture, such as an isocyanate or a poly(carboxylic acid), and the evaporation of the solvent where appropriate.

The polyurethane may itself be obtained in a conventional manner by reacting aromatic and/or aliphatic polyisocyanates with polyester polyols and/or polyether polyols or by the reaction of substantially linear prepolymers having isocyanate ends with polyols as described above.

The polyurethanes may for example be chosen from the group consisting of the polyurethanes of polytetramethylene ether glycol-diphenylmethane diisocyanate (MDI), polytetramethylene ether glycol-tolylene diisocyanate (TDI), polytetramethylene ether glycol-isophorone isocyanate, poly(1,4-oxybutylene) glycol-diphenylmethane diisocyanate (MDI), poly(1,4oxybutylene) glycol-tolylene diisocyanate (TDI), poly(1,4-oxybutylene) glycol-isophorone isocyanate, polyethylene glycol-diphenylmethane diisocyanate (MDI), polyethylene glycol-tolylene diisocyanate (TDI), polyethylene glycol-isophorone isocyanate, polypropylene glycol-diphenylmethane diisocyanate (MDI), polypropylene glycol-tolylene diisocyanate (TDI), polypropylene glycol-isophorone isocyanate, polycaprolactone-diphenylmethane diisocyanate (MDI), polycaprolactone-tolylene diisocyanate (TDI), polycaprolactone-isophorone isocyanate, polyethylene adipate-diphenylmethane diisocyanate (MDI), polyethylene adipate-tolylene diisocyanate (TDI), polyethylene adipate-isophorone isocyanate, polytetramethylene adipate-diphenylmethane diisocyanate (MDI), polytetramethylene adipate-tolylene diisocyanate (TDI), polytetramethylene adipate-isophorone isocyanate, polyethylenepropylene adipate-diphenylmethane diisocyanate (MDI), polyethylene-propylene adipate-tolylene diisocyanate (TDI) and polyethylene-propylene adipate-isophorone isocyanate.

As a variant, the polyurethanes may be chosen from those of formula (1) :

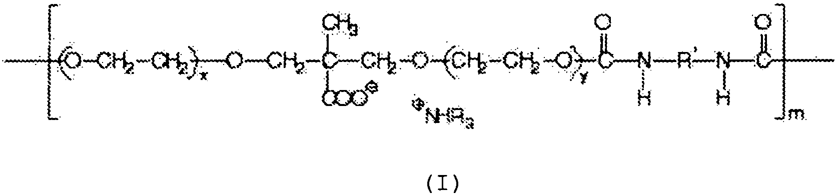

Furthermore, the copolymers of vinylpyrrolidone are preferably copolymers of N-vinylpyrrolidone with a silicone comonomer which is preferably an ethylenically unsaturated monomer, such as (meth)acrylic acid, substituted with at least one silicone chain, such as a polydimethylsiloxane chain. This monomer is advantageously an ester of methacrylic acid and polydimethylsiloxane (dimethicone). Such a copolymer may in particular be obtained by the reaction between an organic silicone complex and a vinylpyrrolidone monomer in an aqueous medium.

The mixing of the two polymers may be carried out in any suitable apparatus, such as a helical screw extruder in the case where they are mixed in the molten state. This mixture may contain from 10 to 80% by weight, preferably from 25 to 60% by weight, of polyurethane, and from 20 to 90% by weight, preferably from 40 to 75% by weight, of vinylpyrrolidone copolymer. It may further contain one or more additives such as fillers, surfactants, pigments, thickeners, perfumes and the like.

The interpolymer obtained may also be termed "complex".

A preferred example of interpolymer which can be used according to the invention is the material having the INCI name PVP/DIMETHICONYLACRYLATE/POLYCARBAMYL/ POLYGLYCOL ESTER, available from the company HYDROMER under the trade name Aquamere^{®} S2011 or from the company PHOENIX under the trade names Pecogel^{®} S2120 and Pecogel^{®} HS-501.

The composition used according to the invention advantageously contains from 0.01 to 5% by weight, preferably from 0.05 to 2% by weight, of interpolymer, relative to the total weight of the composition.

This composition comprises a physiologically acceptable and preferably cosmetically acceptable medium, that is to say which does not have deleterious side effects and in particular which does not produce blotches, chafing, tightness or tingling which are unacceptable for a user of cosmetic products.

This medium comprises at least one oil, and water, which form together a water-in-oil emulsion.

For the purposes of the present invention, the expression "oil" is understood to mean a compound which is liquid at room temperature (25°C) and which, when introduced in an amount of at least 1% by weight into water at 25°C, is not at all soluble in water, or is soluble in an amount of less than 10% by weight, relative to the weight of oil introduced into the water.

As oils, there may be mentioned in particular: linear or branched hydrocarbon oils of inorganic or synthetic origin, synthetic (poly)esters and (poly)ethers and in particular (poly) esters of C₆-C₂₀ acids and C₆-C₂₀ alcohols which are advantageously branched, such as isononyl isononanoate, vegetable oils, branched and/or unsaturated fatty acids, branched and/or unsaturated fatty alcohols, silicone oils, fluorosilicone oils, fluorinated oils, and mixtures thereof.

It is clearly understood that the composition according to the invention may comprise mixtures of the oils mentioned above.

Among these oils, it is preferable that the composition according to the invention contains at least one silicone oil.

The expression "silicone oil" is understood to mean an oil comprising at least one silicon atom, and in particular at least one Si-O group. The silicone oil may be volatile or nonvolatile.

As non-volatile silicone oil, there may be mentioned in particular polydimethylsiloxanes containing at least 8 silicon atoms, polyalkylmethylsiloxanes in which the alkyl chain contains from 8 to 20 carbon atoms and oils identified by the INCI name phenyl trimethicone.

As volatile silicone oil, there may be mentioned in particular certain dimethicones with a viscosity of 5 and 6 cSt, octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane, dodecamethylpentasiloxane, the compounds identified by the INCI names methyl trimethicone and caprylyl methicone and mixtures thereof.

Examples of vegetable oils are in particular wheatgerm oil, sunflower oil, grapeseed oil, sesame oil, maize oil, apricot oil, castor oil, karite oil, avocado oil, olive oil, soybean oil, sweet almond oil, palm oil, rapeseed oil, cottonseed oil, hazelnut oil, macadamia oil, jojoba oil, lucerne oil, poppyseed oil, pumpkin seed oil, sesame oil, gourd oil, rapeseed oil, blackcurrant seed oil, evening primrose oil, millet oil, barley oil, quinoa oil, rye oil, safflower oil, candlenut oil, passion flower oil, musk rose oil or camellia oil.

The expression "hydrocarbon oil" is understood to mean an oil containing solely hydrogen and carbon atoms. Examples of nonvolatile hydrocarbon oils are polybutene, hydrogenated polyisobutene and hydrogenated polydecene. Volatile hydrocarbon oils may be chosen from hydrocarbon oils containing from 8 to 16 carbon atoms, and in particular branched C₈-C₁₆ alkanes (also called isoparaffins) such as isododecane, isodecane or isohexadecane.

The expression "fluorinated oils" is understood to mean an oil containing at least one fluorine atom, such as nonafluoromethoxybutane or perfluoromethylcyclopentane, perfluorodimethylcyclohexane, perfluoroperhydrophenanthrene, perfluorodecalin, and mixtures thereof. without this list being limiting.

The composition used according to the invention may further contain at least one wax and/or at least one lipophilic gelling agent, which are contained in the fatty phase of the emulsion.

The expression "wax" is understood to mean a fatty substance having a melting point greater than 30°C and generally less than 100°C, which is liquid under the conditions for preparing the composition and exhibits in the solid state an anisotropic crystalline organization. Examples of waxes are in particular plant waxes, mineral waxes or synthetic waxes, it being possible for the latter to be advantageously hydrocarbon or silicone waxes. There may thus be mentioned carnauba wax, candelilla wax, rice wax, beeswax (Cera alba), polyethylene waxes which are optionally functionalized, and paraffin wax, as well as ozokerite, microcrystalline waxes, linear C₁₄-C₂₂ fatty alcohols and triesters of C₈-C₂₀ acids and glycerine such as glycerine tribehenate, and mixtures thereof, without this list being limiting. Mention may also be made of acetylated glycol stearate marketed by the company VEVY under the trade name CETACENE^{®}.

Examples of lipophilic gelling agents are in particular silicone polymers and more particularly elastomers of organopolysiloxanes. Among these, there may be mentioned at least partially crosslinked polymers resulting from the reaction of an organopolysiloxane bearing unsaturated groups, such as vinyl or allyl groups, located at the end or in the middle of the chain, preferably on a silicon atom, with another reactive silicone compound such as an organohydrogenopolysiloxane. These polymers are usually available in the form of a gel in a volatile or nonvolatile silicone solvent or in a hydrocarbon solvent. Examples of such elastomers are in particular marketed by the company SHIN ETSU under the trade names KSG-6, KSG-16, KSG-31, KSG-32, KSG-41, KSG-42, KSG-43 and KSG-44, and by the company DOW CORNING under the trade names DC 9040 and DC 9041. Another oily gelling agent consists of a silicone polymer, obtained by self-polymerization of an organopolysiloxane functionalized with epoxy and hydrosilylated groups, in the presence of a catalyst, which is commercially available from the company GENERAL ELECTRIC under the trade name VELVESIL^{®} 125. Another lipophilic gelling agent consists of a cyclic dimethicone/vinyldimethicone copolymer such as that marketed by the company JEEN under the trade name JEESILC^{®} PS (PS-VH, PS-VHLV, PS-CM, PS-CMLV and PS-DM). Another type of lipophilic gelling agent consists of copolymers of styrene and olefins such as ethylene, propylene and/or butylene, optionally combined with silicone or hydrocarbon solvents, as described in particular in application WO 98/38981 and in Patent No. US-6,309,629. They comprise in particular gelling agents based on block terpolymers which are available from the company PENRECO under the trade name VERSAGEL^{®}. Another type of lipophilic gelling agent consists of polyamides such as those identified by the INCI name polyamide-3 and in particular the polymers SYLVACLEAR^{®} AF 1900V and PA 1200V available from the company ARIZONA CHEMICAL and those identified by the INCI name "Ethylenediamine/Hydrogenated Dimer Dilinoleate Copolymer Bis-Di-C14-18 Alkyl Amide" and available for example under the trade name SYLVACLEAR^{®} A200V or SYLVACLEAR^{®} A2614V from the company ARIZONA CHEMICAL. The lipophilic gelling agent may, as a variant, be a bentone or a hydrophobic modified hectorite.

In addition, the aqueous phase of the composition according to the invention may contain one or more hydrophilic gelling agents.

The hydrophilic gelling agent is preferably a hydrocolloid which may be chosen in particular from: homo- and copolymers of acrylic acid and/or of salts or of esters of acrylic acid, such as carbomers, mixtures based on sodium polyacrylate such as GELLINOV^{®}, HYDRILLIEN 9^{®} from lipochemicals and DC RM 2051^{®} from DOW CORNING; homo- and copolymers of acrylamide; homo- and copolymers of acryloylmethylpropanesulphonic acid (AMPS), such as ARISTOFLEX AVC^{®} or HMB^{®} from CLARIANT and SEPINOV EMT^{®} from SEPPIC; polysaccharides such as guar and xanthan gums, carrageenans, grafted levans and nongrafted insulins; and cellulose derivatives, without this list being limiting. The aqueous phase may also contain soluble silicone compounds such as the mixture marketed by the company CLARIANT under the trade name SILCARE^{®} SEA, which has the INCI name "trideceth-9 amodimethicone & trideceth-12".

The composition according to the invention moreover preferably contains from 10 to 50% and more preferably from 20 to 35% by weight of water.

This composition may also contain one or more surfactants, preferably chosen from water-in-oil, advantageously water-in-silicone, emulsifiers such as polyether modified polysiloxanes, in particular oxyethylenated and/or oxypropylenated polydimethylsiloxanes such as those marketed by the company DOW CORNING under the trade name 5225C^{®} Formulation Aid and by the company SHIN-ETSU under the trade name KF-6017^{®}; and the polyethoxylated dipolyhydroxystearate (30 EO) marketed in particular under the trade name ARLACEL^{®} P135 by the company UNIQEMA.

The composition used according to the invention may moreover contain one or more hydrophilic or lipophilic active agents, in particular at least one antiageing active agent which may in particular be chosen from: agents stimulating the expression of tensin 1; agents stimulating the expression of fructosamine-3-kinase or its related protein (FN3K RP); agents stimulating the production of growth factors; anti-glycation agents or deglycating agents; agents increasing the synthesis of collagen or preventing its degradation (anti-collagenase agents, in particular agents inhibiting matrix metalloproteinases); agents increasing the synthesis of elastin or preventing its degradation (anti-elastase agents); agents increasing the synthesis of glycosaminoglycans or of proteoglycans or preventing their degradation (anti-proteoglycanase agents); agents stimulating the synthesis of integrins by the fibroblasts; agents increasing the proliferation or differentiation of the keratinocytes; agents increasing the proliferation of the fibroblasts; anti-oxidant or anti-free-radical or anti-pollution agents; and mixtures thereof, without this list being limiting.

Examples of such agents are in particular: plant extracts and in particular extracts of *Butea frondosa, Magnolia champaca, Vanilla planifolia, Cedrus atlantica, Canarium commune* (elemi), *Zingiber cassumunar Roxb., Chondrus crispus, Thermus thermophilus, Pisum sativum, Centella asiatica,* Scenedesmus, *Moringa pterygosperma,* hamamelis, *Castanea sativa, Hibiscus sabdriffa, Polyanthes tuberosa, Argania spinosa, Aloe vera, Narcissus tarzetta,* or liquorice; an essential oil of *Citrus aurantium* (Neroli); α-hydroxyacids such as glycolic, lactic and cyclic acids, and their esters ; β-hydroxyacids, such as salicylic acid and its derivatives; hydrolysates of plant proteins (in particular of soybean or of hazelnut); acylated oligopeptides; extracts of yeast, and in particular of *Saccharomyces cerevisiae;* extracts of algae, and in particular of laminaria; vitamins and their derivatives such as retinyl palmitate, ascorbic acid, ascorbyl glucoside, magnesium or sodium ascorbyl phosphate, ascorbyl palmitate, ascorbyl tetraisopalmitate, ascorbyl sorbate, tocopherol, tocopheryl acetate and tocopheryl sorbate; and mixtures thereof.

The composition used according to the invention may further contain humectants such as hyaluronic acid and its salts and/or polyols such as glycerine.

It further advantageously contains at least one filler. This term is understood to mean any particle of any shape (in particular spherical or lamellar), which is inorganic or organic, and insoluble in the composition. Examples of fillers are talc, mica, silica, kaolin, boron nitride, starch, starch modified with octenylsuccinic anhydride, polyamides, silicone resins, powders of silicone elastomers and powders of acrylic polymers, in particular poly(methyl methacrylate). The fillers may in particular consist of several layers of different chemical nature and/or physical form, and may in particular be provided in the form of leaflets coated with spherical fillers. They may be modified with the aid of various surface treatments. One example of a surface-treated filler consists of silica modified with an ethylene/methacrylate copolymer, marketed in particular by the company KOBO under the trade names DSPCS 20N-I2^{®}, DSPCS/3H-I2^{®} and DSPCS-I2^{®}.

The composition may also contain at least one dye substance chosen from water-soluble or fat-soluble dyes, fillers having the effect of colouring and/or opacifying the composition and/or the skin, such as pigments, pearlescent agents, lacquers (water-soluble dyes adsorbed onto an inert mineral support) and mixtures thereof. These dye substances may be optionally surface-treated with an hydrophobic agent such as silanes, silicones, fatty acid soaps, C₉-C₁₅ fluoroalcohol phosphates, acylate/dimethicone copolymers, C₉-C₁₅ fluoroalcohol phosphate/silicone mixed copolymers, lecithins, carnauba wax, polyethylene, chitosan, alumina and optionally acrylated amino acids such as lauroyllysine, disodium stearoyl glutamate and aluminium acyl glutamate. The pigments may be mineral or organic, natural or synthetic. Examples of pigments are in particular iron, titanium, chromium or zinc oxides, ultramarine blue, Prussian blue, carbon black, and composite pigments and goniochromatic, pearlescent, interference, photochromic or thermochromic pigments, without this list being limiting. The pearlescent agents may be chosen from those conventionally present in makeup products, such as micatitanium dioxide.

The composition according to the invention may also contain antioxidants such as the alkylated or phosphorylated esters of ascorbic acid, or tocopherol and its esters; sequestrants such as EDTA salts; pH regulators; preservatives such as parabens and phenoxyethanol; and perfumes.

The composition may further contain at least one UV-screening agent chosen from organic and inorganic screening agents and mixtures thereof. As organic screening agents, there may be mentioned in particular dibenzoylmethane derivatives (including butylmethoxydibenzoylmethane), cinnamic acid derivatives (including ethylhexyl methoxycinnamate), salicylates, paraaminobenzoic acids, β,β'-diphenylacrylates, benzophenones, benzylidenecamphor derivatives, phenylbenzimidazoles, triazines, phenylbenzotriazoles and anthranilic derivatives. As inorganic screening agents, there may be mentioned in particular screening agents based on inorganic oxides in the form of pigments or nanopigments, and in particular based on titanium dioxide or zinc oxide which may be coated or uncoated, for example with the aid of dimethicone, acyl glutamates, silica, alumina and/or aluminium stearate.

The total quantity of oily compounds contained in the composition used according to the invention preferably represents from 30 to 50% by weight, relative to the total weight of this composition. It is understood that they include, in addition to the oils listed above, other constituents mentioned above, such as, for example, UV-screening agents and W/O emulsifiers in liquid form.

The composition used according to the invention may be a care or makeup composition for the skin, in particular a care fluid, serum or cream or a foundation.

It may be applied to the skin of the face and optionally of the neck and/or of the neck and shoulders for smoothing wrinkles and fine lines. The method according to the invention is generally carried out on a wrinkled and/or flabby skin.

The invention will be understood more clearly in the light of the following nonlimiting examples which are given solely by way of illustration.

### EXAMPLES

### Example 1 : Cosmetic composition

### Example I-A : Foundation

A composition containing the constituents identified below by their function and in capitals by their INCI name (with reference to the CTFA Dictionary, 11th Edition, 2006) in the percentages by weight indicated opposite these constituents, was prepared in a manner conventional for persons skilled in the art.

| **Constituent** | **%** |
|---|---|
| CYCLOPENTASILOXANE & C30-45 ALKYL CETEARYL DIMETHICONE CROSSPOLYMER | 3.00 |
| PEG-10 DIMETHICONE | 5.00 |
| Silicone oils | 23.00 |
| Vegetable oil | 0.50 |
| Antioxidant | 0.20 |
| Fat-soluble organic UV-screening agent | 7.50 |
| Cultured inorganic UV-screening agents | 7.00 |
| Preservative | 3.00 |
| CYCLOPENTASILOXANE & DISTEARDIMONIUM HECTORITE & SD ALCOHOL 40 | 1.00 |
| Cultured pigments | 9.15 |
| Pearlescent agent | 1.00 |
| Fillers | 5.85 |
| GLYCERIN | 2.00 |
| BUTYLENE GLYCOL | 2.00 |
| SODIUM CHLORIDE | 1.00 |
| PVP/DIMETHICONYLACRYLATE/POLYCARBAMYL/ POLYGLYCOL ESTER | 0.50 |
| Water-soluble active agents | 8.40 |
| Water | qs 100.00 |

### Example 1-B: Anti-wrinkle serum

The following composition is prepared:

| **Constituents** | **%** |
|---|---|
| Alcohol | 3.000 |
| Fillers | 7.500 |
| Volatile silicone | 12.500 |
| HYDROGENATED CASTOR OIL | 0.250 |
| CYCLOPENTASILOXANE & PEG/PPG-18/18 DIMETHICONE | 9.000 |
| Co-emulsifier | 0.500 |
| Antioxidant | 0.025 |
| CYCLOPENTASILOXANE & C30-45 ALKYL CETEARYL DIMETHICONE CROSSPOLYMER | 15.000 |
| Fatty ester | 1.000 |
| PEG-45 DODECYL GLYCOL COPOLYMER | 1.000 |
| Glycerine | 1.500 |
| Propylene glycol | 1.500 |
| Sequestrant | 0.05 |
| Sodium chloride | 0.800 |
| Preservatives | 0.535 |
| Gum | 0.500 |
| Ethyl phthalate | 0.030 |
| Dye | 0.200 |
| FRAGRANCE | 0.400 |
| Active agents | 10.510 |
| PVP/DIMETHICONYLACRYLATE/POLYCARBAMYL/ POLYGLYCOL ESTER | 0.600-1.600 |
| Water | qs 100 |

### Example 2: Sensory evaluation

Composition 1-A of Example 1 was tested by 20 subjects of the Caucasian type showing signs of skin ageing (wrinkles, fine lines, lack of firmness). To do this, the composition was applied to the whole of the face and the neck, at the rate of once by day, every morning for six weeks.

Each subject completed a questionnaire containing a number of descriptors relating to the cosmetic qualities of the product and to the makeup result obtained after application.

76% of the subjects described a sensation of "drawn features" and a "lifted" appearance of the skin immediately after application of the product. Furthermore, 76% of the subjects judged that the tightening effect remained perceptible several hours after the application.

These results show that the emulsions according to the invention make it possible to tighten the skin immediately in a perceptible and lasting manner.

## Claims

1. Cosmetic method for tightening the skin and/or smoothing wrinkles and fine lines by a tightening effect, comprising the application to the skin of a composition in the form of a water-in-oil emulsion containing, in a physiologically acceptable medium, an interpolymer comprising at least one polyurethane and at least one copolymer of vinylpyrrolidone and a silicone monomer.

2. Method as claimed in Claim 1, **characterized in that** the silicone monomer is an ethylenically unsaturated monomer substituted with at least one silicone chain such as a polydimethylsiloxane chain.

3. Method according to Claim 2, **characterized in that** the silicone monomer is a (meth)acrylic acid ester.

4. Method according to any of Claims 1 to 3, **characterized in that** the composition contains from 0.01 to 5% by weight, preferably from 0.05 to 2% by weight, of interpolymer, relative to the total weight of the composition.

5. Method according to any one of Claims 1 to 4, **characterized in that** the composition contains at least one silicone oil.

6. Method according to any one of Claims 1 to 5, **characterized in that** the total quantity of the oily compounds contained in the composition represents from 30 to 50% by weight, relative to the total weight of this composition.

7. Method according to any one of Claims 1 to 6, **characterized in that** it is used on a wrinkled and/or flabby skin.

## Patentansprüche

1. Kosmetisches Verfahren zur Straffung der Haut und/oder zur Glättung von Falten und feinen Linien durch eine Straffungswirkung, umfassend die Auftragung einer Zusammensetzung in Form einer Wasser-in-Öl-Emulsion, die in einem physiologisch annehmbaren Medium ein Interpolymer, das wenigstens ein Polyurethan umfasst, und wenigstens ein Copolymer von Vinylpyrrolidon und einem Silikonmonomer enthält, auf die Haut.

2. Verfahren gemäß Anspruch 1, **dadurch gekennzeichnet, dass** das Silikonmonomer ein ethylenisch ungesättigtes Monomer ist, das mit wenigstens einer Silikonkette, wie einer Polydimethylsiloxan-Kette, substituiert ist.

3. Verfahren gemäß Anspruch 2, **dadurch gekennzeichnet, dass** das Silikonmonomer ein (Meth)acrylsäureester ist.

4. Verfahren gemäß einem der Ansprüche 1 bis 3, **dadurch gekennzeichnet, dass** die Zusammensetzung 0,01 bis 5 Gew.-%, vorzugsweise 0,05 bis 2 Gew.-%, Interpolymer enthält, bezogen auf das Gesamtgewicht der Zusammensetzung.

5. Verfahren gemäß einem der Ansprüche 1 bis 4, **dadurch gekennzeichnet, dass** die Zusammensetzung wenigstens ein Silikonöl enthält.

6. Verfahren gemäß einem der Ansprüche 1 bis 5, **dadurch gekennzeichnet, dass** die Gesamtmenge der in der Zusammensetzung enthaltenen öligen Verbindungen 30 bis 50 Gew.-% beträgt, bezogen auf das Gesamtgewicht dieser Zusammensetzung.

7. Verfahren gemäß einem der Ansprüche 1 bis 6, **dadurch gekennzeichnet, dass** es auf einer faltigen und/oder schlaffen Haut angewendet wird.

## Revendications

1. Procédé cosmétique pour retendre la peau et/ou lisser les rides et ridules par effet tenseur, comprenant l'application sur la peau d'une composition sous la forme d'une émulsion eau-dans-huile renfermant, dans un milieu physiologiquement acceptable, un interpolymère comprenant au moins un polyuréthane et au moins un copolymère de vinylpyrrolidone et d'un monomère siliconé.

2. Procédé selon la revendication 1, **caractérisé en ce que** le monomère siliconé est un monomère à insaturation éthylénique substitué par au moins une chaîne siliconée telle qu'une chaîne polydiméthylsiloxane.

3. Procédé selon la revendication 2, **caractérisé en ce que** le monomère siliconé est un ester d'acide (meth)acrylique.

4. Procédé selon l'une quelconque des revendications 1 à 3, **caractérisé en ce que** la composition renferme de 0,01 à 5% en poids, préférentiellement de 0,05 à 2% en poids, d'interpolymère, par rapport au poids total de la composition.

5. Procédé selon l'une quelconque des revendications 1 à 4, **caractérisé en ce que** la composition renferme au moins une huile de silicone.

6. Procédé selon l'une quelconque des revendications 1 à 5, **caractérisé en ce que** la quantité totale des composés huileux contenus dans la composition représente de 30 à 50 % en poids, par rapport au poids total de cette composition.

7. Procédé selon l'une quelconque des revendications 1 à 6, **caractérisé en ce qu'**il est mis en oeuvre sur une peau ridée et/ou relâchée.
